# EUROPEAN PATENT APPLICATION

(11) **EP 4 492 066 A1**
(43) Date of publication of application: **15.01.2025**
(21) Application number: 23766018.8
(22) Date of filing: 07.03.2023
(51) Int. Cl.: G01N 35/02, G01N 35/00, G01N 21/64, G01N 33/53

(54) **CONFOCAL LASER TECHNOLOGY-BASED FULLY AUTOMATED IMMUNOASSAY ANALYZER AND DETECTION METHOD THEREOF**

(30) Priority: 08.03.2022 CN 202210220350
(71) Applicant: Shanghai Saga Biotech Co., Ltd., Shanghai 201203 (CN)
(72) Inventor: ZHANG, Xu, Shanghai 201203 (CN); XU, Yingyuan, Shanghai 201203 (CN)
(74) Representative: Rondano, Davide
(86) International application number: PCT/CN2023/080160
(87) International publication number: WO 2023/169440

(57) **Abstract**

A confocal laser technology-based immunoassay analyzer and a detection method thereof. The immunoassay analyzer comprises: an X-direction motion module (17), a reagent strip module (6), a Z-direction motion module (7), an operation arm module (2), a magnetic attraction module (4), and an optical motion detection module (9). The detection method comprises: (1) attractioning a sample and alysates; (2) mixing and diluting the sample and the lysates; (3) mixing the mixed solution with a magnetic particle solution; (4) performing constant-temperature incubation and separating magnetic particles from a reaction liquid; (5) cleaning the magnetic particles; (6) mixing magnetic beads and the reaction liquid and placing a mixture into a reaction tank; and (7) completing detection and signal output of the reaction liquid.

## Description

### Technical field

The present invention belongs to the field of biological reagents and relates to a confocal laser technology-based fully automated immunoassay analyzer, as well as a detection method thereof.

### Technical Background

Fluorescence immunoassay and chemiluminescence are two types of molecular luminescence spectroscopy anacracking. The former has the advantages of stable signal and controllable luminescence. The substance to be tested only emits fluorescence after excitation, and the intensity of fluorescence is proportional to the concentration of the substance to be tested. Traditional fluorescence immunoassay works in the visible light spectral range of 400-600nm, but its weakness is high background noise and low detection sensitivity. The latter is based on the principle of linear quantitative relationship between the concentration of the substance to be tested in the chemical detection system and the chemiluminescence intensity of the system under certain conditions. The instrument is used to detect the luminescence intensity of the substance to be tested and determine the content of the substance to be tested. It is categorized as indirect chemiluminescence method, direct chemiluminescence method, and electrochemical luminescence method, etc. The disadvantages of indirect chemiluminescence method and direct chemiluminescence method is that the luminescence is uncontrollable and the signal stability is poor. The electrochemical luminescence method emits controllable light, but there is cross contamination when the detection pool is shared and not well controlled.

Chemiluminescence immunoassay combines highly sensitive chemiluminescence assay technology with highly specific immune reactions, which is used for the detection and analysis of anacrackinging various antigens, antibodies, hormones, enzymes, vitamins, and drugs. It is an immunoassay technology developed after radioimmunoassay, enzyme immunoassay, fluorescence immunoassay, and time-resolved fluorescence immunoassay. Chemiluminescence method has the advantages of high sensitivity, strong specificity, high accuracy, and wide detection range. Compared to semi quantitative enzyme-linked immunosorbent assay, chemiluminescence is truly quantitative and has a faster and more convenient detection speed. At the same time, the chemiluminescent markers are stable and the reagent has a long shelf life, greatly facilitating the needs of clinical applications.

### Summary of invention

The purpose of this application is to provide a confocal laser technology-based fully automated immunoassay analyzer. By combining laser confocal technology with near-infrared fluorescence technology, the problem of unstable luminescence signals in indirect and direct chemiluminescence methods is solved, and the problem of cross contamination in the shared detection pool of electrochemical luminescence methods is also solved. It not only retains the advantages of strong signal stability in fluorescence immunoassay, but also possesses the advantages of high sensitivity and wide linear range of chemiluminescence, making it a perfect combination of the two methods.

The present application discloses a confocal laser technology-based fully automated immunoassay analyzer. The instrument conprises: an X-direction motion module, a reagent strip module, a Z-direction motion module, an operating arm module, a magnetic attraction module, and an optical motion detection module, wherein the X-direction motion module comprises a rubber foot, a large base plate, an X direction screw motor, a motor fixing seat, a wide guide rail, and the first connecting block, wherein the first connecting block is connected to the slider of the wide guide rail and the nut of the X-direction screw motor. When the X-direction screw motor rotates, The first connecting block can be moved along the sliding rail direction of the wide guide rail; the reagent strip module includes a reagent strip fixing plate, a tube head frame, a waste liquid tank, a reagent strip, and a heating rod, wherein the reagent strip is connected to the first connecting block of the fixed plate and the X-direction motion module; The Z-direction motion module comprises a connecting plate, a slant support, a vertical column, an upper fixing plate, a lower fixing plate, a Z-axis screw motor, a guide column, a sliding block, a guide column fixing seat, a connecting arm, and a shaft blocking vertical column. The z-axis screw motor connects the guide column sliding block through the connecting arm, the guide column sliding block can slide up and down along the guide column, and the shaft blocking vertical column is fixed on the lower fixing plate: the operating arm module comprises a discharging gun, a TIP withdrawing structure, an injection pump, and a PTFE pipe; The magnetic attraction module comprises a fixed plate, a magnetic attraction motor, gears, racks, linear slide rails, a magnetic attraction plate, and a cylindrical magnet. The magnetic attraction module is installed in the area below the Z-direction motion module and above the reagent strip fixed plate. The magnetic attraction motor is installed on the fixed plate, which is equipped with gears. The magnetic attraction plate is installed on the sliding block of the slide rail. The optical motion detection module includes a crossbeam, a Y-direction through-axis motor, a motor base, a connecting block 2, a linear slide rail, a manual fine-tuning table and a optical detector.

In a preferred example, one end of the discharging gun is used to load the TIP head, the other end of the discharging gun is connected to the injection pump through a PTFE tube, which can aspirate and inject the test solution.

In a preferred example, there are two sets of linear sliding rails installed on the fixed plate in the magnetic attraction module.

In a preferred example, the magnetic attraction plate is equipped with a rack and 8 circular magnets are installed at the ends.

In a preferred example, the motion range of the optical motion detection module covers all reagent strips on the reagent strip fixing plate.

In a preferred example, the optical detector is connected to the second connecting block through a manual fine-tuning table.

In a preferred example, the operating arm module is connected to the slider of the Z-direction motion module and can move vertically.

In a preferred example, the second connecting block is fixed on the sliding rail slider and connected to the motor. When the motor is operating, the second connecting block 2 can move left and right along the sliding rail.

In a preferred example, the crossbeam is fixed on the column of the Z-direction motion module, which is equipped with a linear sliding rail and a Y-direction through axis motor.

The present application also discloses a near-infrared fluorescence immunoassay method based on laser confocal technology, characterized in that the method is suitable for a small fully automated immunoassay analyzer as claimed in claim 1, and comprises the following steps:
(1) Aspirate the sample and lysates:
   The Z-axis screw motor lifts the discharging gun, while the X-axis screw motor transports the sample slot below the TIP head. The Z-axis v screw motor then lowers the TIP head into the sample slot. The injection pump provides negative pressure to the TIP head through the PTFE tube, and the TIP head aspirates the sample liquid; Repeat the above steps, lower the TIP head into the cracking reagent tank, and aspirate the lysates:
(2) Mixing and dilution of samples and lysates:
   The X-axis screw motor moves the diluent reagent tank below the TIP head, while the Z-axis screw motor lowers the TIP head into the diluent reagent tank. The injection pump provides 4positive pressure to inject the sample and lysates into the diluent, while the injection pump provides negative pressure to aspirate the mixture into the TIP head. The injection pump then provides positive pressure to inject the mixture into the diluent reagent tank. Repeat this process several times to complete the mixing and dilution of the sample and lysates:
(3) Mix the mixture with magnetic particle liquid:
   The injection pump provides negative pressure and draws a certain volume of diluent; The Z-axis screw motor lifts the TIP head, while the X-axis screw motor moves the magnetic particle solution reagent tank below the TIP head. The Z-axis screw motor lowers the TIP head into the magnetic particle solution, and the injection pump provides positive pressure to inject the mixture of sample and lysates into the magnetic particle solution. The injection pump sequentially provides negative pressure and positive pressure several times to fully mix the aforementioned mixture:
(4) Constant temperature incubation and separation of magnetic particles from reaction solution:
   Connect the power to the two heating rods and keep the incubation tank **in** a certain constant temperature environment; after incubating at a constant temperature for a certain period of time, the injection pump provides negative pressure to aspirate all the magnetic particle liquid, and the Z-direction screw motor lifts the TIP head; The magnetic rod motor drives the magnetic rod to move forward and approach the sidewall of the TIP head: after a certain period of time, the magnetic particles are completely adsorbed on the inner wall of the TIP head. The injection pump provides positive pressure to inject the reaction solution from the TIP head into the magnetic particle liquid reagent tank, completing the separation of the magnetic particles and the reaction solution;
(5) Cleaning magnetic particles:
   The X-axis screw motor moves the first cleaning solution tank of the reagent strip below the TIP head. The magnetic rod motor temporarily moves the magnetic rod away, and the Z-axis motor lowers the TIP head into the cleaning solution. The injection pump provides negative pressure to aspirate the cleaning solution, and then the injection pump provides positive pressure to discharge the cleaning solution from the TIP head. This process is repeated for a certain period of time. Then, the injection pump provides negative pressure to aspirate the cleaning solution, and the Z-axis motor lifts the TIP head, The magnetic rod motor moves the magnetic rod close to the sidewall of the TIP head and slowly discharges the cleaning solution. Repeat the above steps and complete the subsequent cleaning work in the second and third cleaning solution tanks respectively:
(6) Mix the magnetic beads and substrate solution and place them in the reaction tank:
   The Z-axis screw motor lifts the TIP head, while the X-axis screw motor moves the reaction tank of the reagent strip below the TIP head. The Z-axis screw motor lowers the TIP head into the reaction tank, and the injection pump provides positive repulsion to inject the substrate solution into the reaction tank. The injection pump then provides negative pressure and positive pressure several times, completing the mixing of the magnetic beads and substrate solution;
(7) completes the detection and signal output of the reaction solution:
   Move the reagent strip reaction tank to the lower part of the objective lens of the optical motion detection module towards the screw motor, turn on the excitation light source, and the optical detector will convert the received near-infrared fluorescence signal into an electrical signal output; The y-axis through axis motor moves to move the detection module sequentially above the reaction tanks of the other seven sets of reagent strips, completing the detection and signal output of the other seven sets of reaction liquids.
   The specification of this application contains a large number of technical features distributed among various technical solutions. If it is necessary to list all possible combinations of technical features (i.e. technical solutions) of this application, it will make the specification too lengthy. In order to avoid this problem, the various technical features disclosed in the above invention content, the various technical features disclosed in the following embodiments and examples, and the various technical features disclosed in the drawings can be freely combined with each other to form various new technical solutions (these technical solutions should be considered as already recorded in this specification), unless the combination of such technical features is technically infeasible. For example, in one example, feature A ten B ten C is disclosed, and in another example, feature A+B+D ton is disclosed, and features C and D are equivalent technical means that play the same role. Technically, only one can be used and cannot be used at the same time. Feature E can be combined with feature C technically. Therefore, the solution of A+B+C+D should not be considered as already recorded due to its technical infeasibility, while the solution of A+B+C+E should be considered as already recorded.

### Drawings

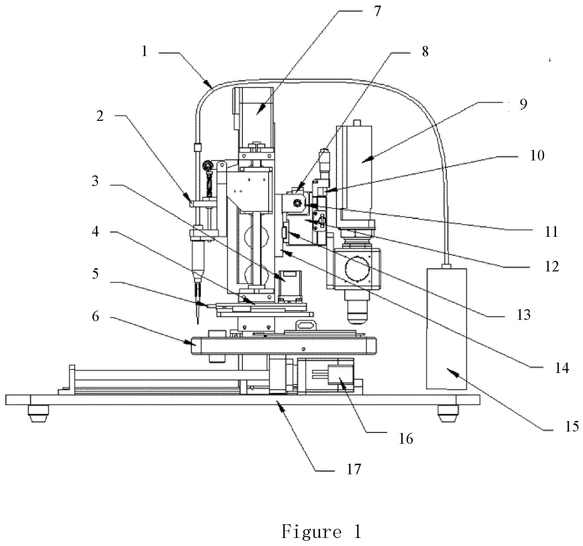

Figure 1 is a side view of the tiny fully automated immunoassay e analyzer of the present invention;

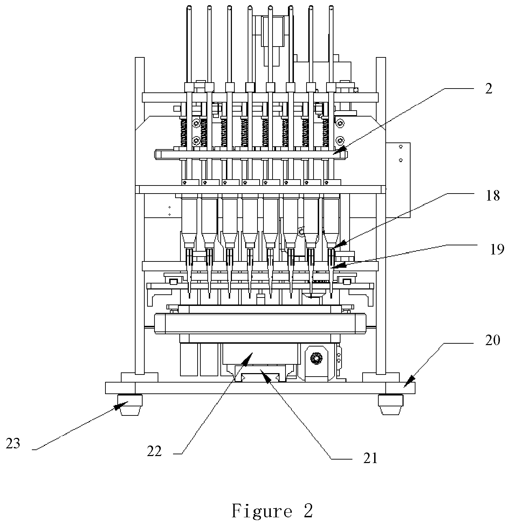

Figure 2 is a front view of the tiny fully automated immunoassay analyzer of the present invention;

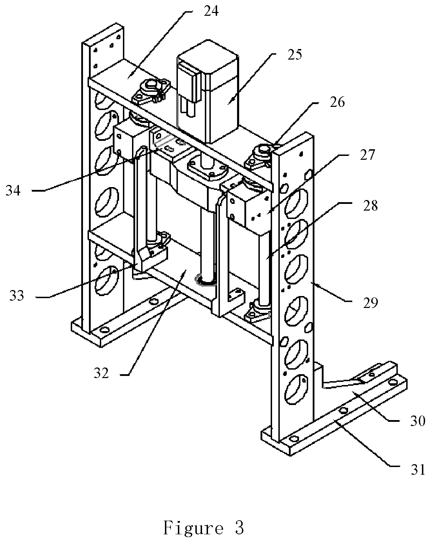

Figure 3 is a schematic diagram of the Z-axis motion module of the tiny fully automated immunoassay analyzer according to the present invention;

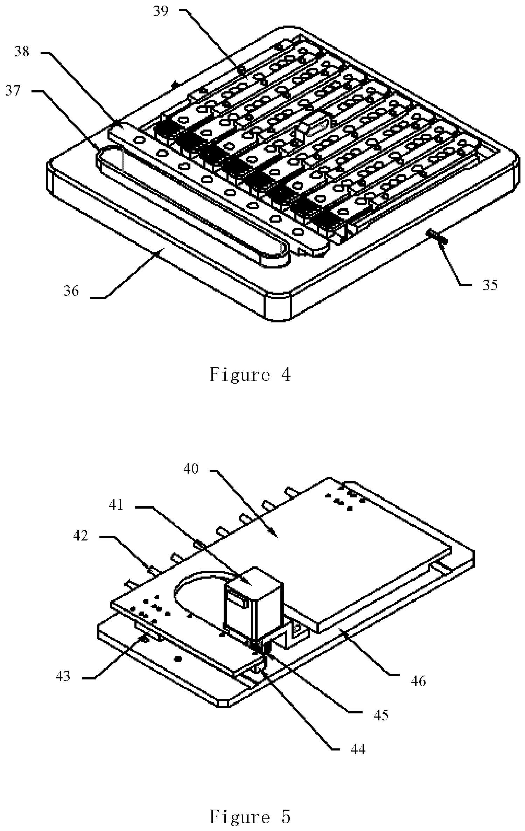

Figure 4 is a schematic diagram of the reagent strip module of the tiny fully automated immunoassay analyzer of the present invention;
Figure 5 is a schematic diagram of the magnetic attraction module of the tiny fully automated immunoassay analyzer according to the present invention:;

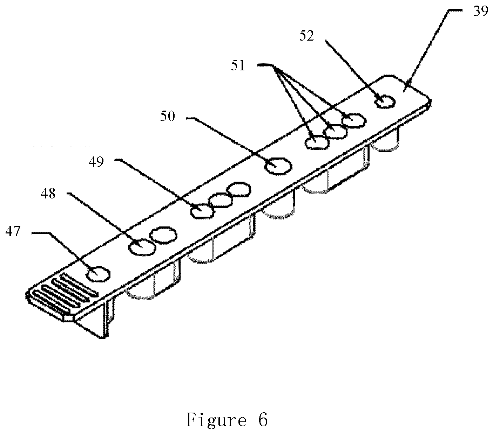

Figure 6 is a schematic diagram of the reagent strip components in the reagent strip module of the tiny fully automated immunoassay analyzer according to the present invention;

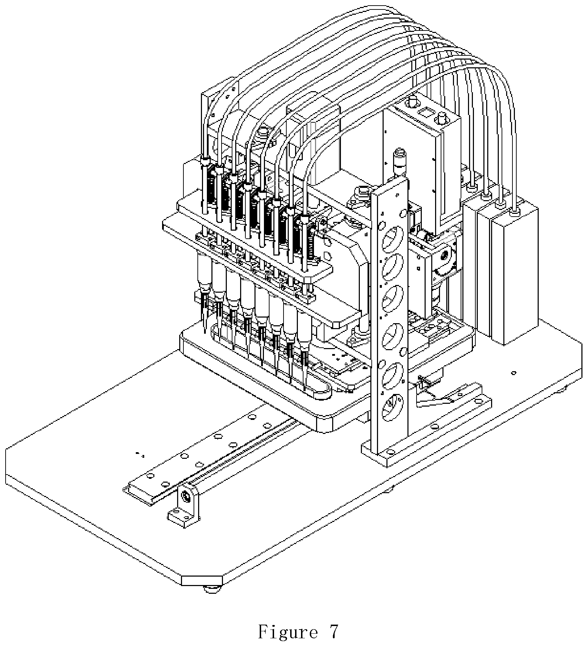

Figure 7 is a stereogram of the tiny fully automated immunoassay analyzer of the present invention;

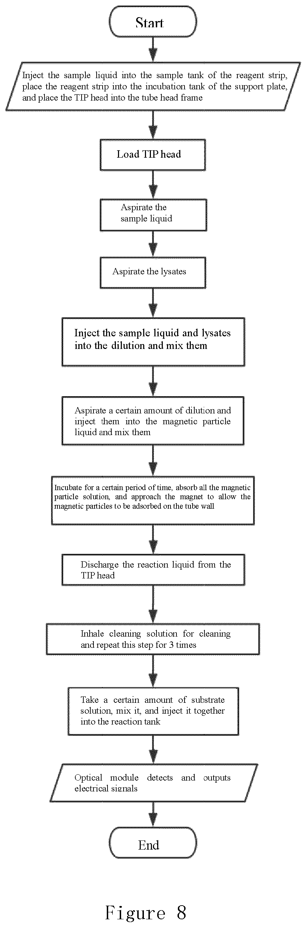

Figure 8 is a flowchart of the detection method according to the present invention.

### Explanation of attached image markings:

1- PTFE pipe; 2- operating arm module; 3- magnetic rod motor; 4- magnetic attraction module; 5- magnetic rod; 6- reagent strip module; 7- Z-direction motion module; 8- Y-axis through axis motor; 9- Optical motion detection module; 10- manual fine-tuning table; 11- motor base; 12- the second connecting block; 13- linear slide rail; 14- Cross beam; 15-injection pump; 16- X-direction screw motor; 17- X-direction motion module; 18-discharging gun; 19-TIP head; 20-Large bottom plate; 21-wide slide rail; 22- the first connecting block; 23- foot; 24- upper fixing plate; 25- Z-axis screw motor; 26- guide column fixing seat; 27- slider; 28- guide column; 29- Column; 30- diagonal bracing; 31-connecting plate; 32-lower fixing plate; 33- blocking shaft column; 34- connecting arm; 35- heating rod; 36- reagent strip fixing plate; 37- waste liquid tank; 38- pipe head frame; 39- reagent strip; 40- magnetic attraction plate; 41- magnetic attraction motor; 42- columnar magnet; 43- linear slide rail; 44- rack; 45- gear; 46-fixing plate; 47-sample tank; 48- lysates tank; 49- dilution tank 50- magnetic particle liquid tank; 51- cleaning liquid tank; 52- reaction tank.

### Detailed description

The inventor of the present invention has developed for the first time for a confocal laser technology-based fully automated immunoassay analyzer. By combining laser confocal technology with near-infrared fluorescence technology, the problem of unstable luminescence signals in indirect and direct chemiluminescence methods is solved, and the problem of cross contamination in the shared detection pool of electrochemical luminescence methods is also solved. It not only retains the advantages of strong signal stability in fluorescence immunoassay, but also has the advantages of high sensitivity and wide linear range in chemiluminescence anacracking.

The advantages of the present invention are:
(1)The immunoassay analyzer involved in this application can be used for immune anacracking of serum and whole blood. When detecting whole blood samples, it can maintain extremely high detection sensitivity and stability, and the reaction time is short. It can complete the detection of 8 samples in 20 minutes
(2) The reagent strip matched with the immunoassay analyzer adopts an integrated reagent strip, which is pre packaged with luminescent liquid, magnetic bead liquid, fluorescein, etc. It can be used imeediately when it is unpacked , greatly simplifying the design of the instrument, improving its reliability and convenience of use
(3) The immunoassay analyzer referred to in this application adopts laser confocal technology, and the excitation light wavelength and the received fluorescence wavelength are both in the near-infrared band of 700-900nm, overcoming the weakness of the original fluorescence immunoassay analyzer with high background, maintaining the advantages of stable signal of the fluorescence immunoassay analyzer, and having the sensitivity and linear range of chemiluminescence.

In order to make the purpose, technical solution, and advantages of this application clearer, a further detailed description of the implementation method of this application will be provided below in conjunction with the accompanying drawings.

### Embodiment

The present invention discloses a tiny fully automated immunoassay analyzer, comprising an X-direction motion module 17, a reagent strip module 6, a Z-direction motion module 7, an operating arm module 2, a magnetic attraction module 4, and an optical motion detection module 9.

Wherein, the X-direction motion module 17 comprises a rubber foot 23, a large bottom plate 20, a X-direction screw motor 16, motor fixing seat, wide slide rail and the first connecting block 22. In an embodiment, the first connecting block 22 is connected to the slider 27 of the wide guide rail and the nut of the X-direction screw motor 16. When the X-direction screw motor 16 rotates, the connecting block 22 can move along the sliding direction of the wide guide rail.

The reagent strip module 6 comprises a reagent strip fixing plate 36, a tube head frame 38, a waste liquid tank 37, a reagent strip 39, and a heating rod 35. In one embodiment, the reagent strip fixing plate 36 is connected to the first connecting block 22 of the X-axis motion module 17.

The Z-direction motion module 7 comprises a connecting plate 31, a diagonal bracing 30, a vertical column 29, an upper fixing plate 24, a lower fixing plate 32, a Z-axis screw motor 25, a guide column 28, a slider 27, a guide column fixing seat 26, a connecting arm 34, and a shaft blocking vertical column 29. The Z-axis screw motor 25 connects a guide column slider 27 through the connecting arm 34. The guide column slider 27 can slide up and down along the guide column, and the shaft blocking vertical column 29 is fixed on the lower fixing plate 32:

The operating arm module 2 includes a discharging gun 18, a return TIP structure, an injection pump 15, and a PTFE tube 1. In one embodiment, one end of the discharging gun 18 is used for loading the TIP head 19, and the other end is connected to the injection pump 15 through the PTFE tube 1, which can aspirate and inject the test solution. In another embodiment, the operating arm module 2 is connected to the slider 27 of the Z-direction motion module 7 and can move vertically.

The magnetic attraction module 4 includes a fixing plate 46, a magnetic attraction motor 41, a gear 45, a rack 44, a linear slide rail 43, a magnetic attraction plate 40, and a columnar magnet 42. In one embodiment, the magnetic attraction module 4 has two sets of linear slide rails 43 installed on the fixing plate 46, and the magnetic attraction module 4 is installed below the Z-direction motion module 7 and in the area of the reagent strip fixing plate 36. The magnetic attraction plate 40 is equipped with a rack 44, and its end is equipped with 8 circular magnets, The magnetic attraction motor 41 is installed on a fixed plate 46, which is equipped with a gear 45, and the magnetic attraction plate 40 is installed on the sliding rail slider 27.

The optical motion detection module 9 includes a crossbeam 14, a Y-axis penetrating motor 8, a motor base 11, the second connecting block 12, a linear slide rail 13, a manual fine-tuning table 10, and an optical detector. In one embodiment, the optical detector is connected to the second connecting block 12 through the motion fine-tuning stage 10. The second connecting block 12 is fixed on the slide rail slider 27 and connected to the motor. When the motor is in operation, the second connecting block 12 can move left and right along the slide rail. The crossbeam 14 is fixed on the column 29 of the Z-direction motion module 7, which is equipped with a linear slide rail 13 and a Y-direction through axis motor 8. The movement range of the optical motion detection module 9 covers all reagent strips 39 on the reagent strip fixing plate 36.

When using, perform near-infrared fluorescence immunoassay by the following steps:
(1) Aspirate the sample and lysateslysates
   The Z-axis screw motor 25 lifts the discharging gun 18, while the X-axis screw motor 16 transports the sample slot 47 below the TIP head 19. The Z-axis screw motor 25 then lowers the TIP head 19 into the sample slot 47. The injection pump 15 provides negative pressure to the TIP head 19 through the PTFE tube 1, and the TIP head 19 aspirates the sample liquid; Repeat the above steps, lower the TIP head 19 into the cracking reagent tank 48 and aspirate the lysates:
   (2) Mixing and dilution of samples and lysates:
   The X-axis screw motor 16 moves the diluent reagent tank 49 below the TIP head 19, while the Z-axis screw motor 25 lowers the TIP head 19 into the diluent reagent tank 49. The injection pump 15 provides positive pressure to inject the sample and lysates into the diluent, while the injection pump 15 provides negative pressure to aspirate the mixture into the TIP head 19. The injection pump 15 then provides positive pressure to inject the mixture into the diluent reagent tank 49. Repeat this process several times to complete the mixing and dilution of the sample and lysates:
   (3) Mix the mixture with magnetic particle liquid:
   The injection pump 15 provides negative pressure and draws a certain volume of diluent; The Z-axis screw motor 25 lifts the TIP head 19, while the X-axis screw motor 16 moves the magnetic particle solution reagent tank 50 below the TIP head 19. The Z-axis screw motor 25 lowers the TIP head 19 into the magnetic particle solution, and the injection pump 15 provides positive pressure to inject the mixture of sample and lysates into the magnetic particle solution. The injection pump 15 sequentially provides negative pressure and positive pressure several times to fully mix the aforementioned mixture:
   (4) Constant temperature incubation and separation of magnetic particles from reaction solution:
   Connect the power to the two heating rods 35 and keep the incubation tank in a certain constant temperature environment; after incubating at a constant temperature for a certain period of time, the injection pump 15 provides negative pressure to aspirate all the magnetic particle liquid, and the Z-direction screw motor 25 lifts the TIP head 19; The magnetic rod motor 3 drives the magnetic rod 5 to move forward and approach the sidewall of the TIP head 19: after a certain period of time, the magnetic particles are completely adsorbed on the inner wall of the TIP head 19. The injection pump 15 provides positive pressure to inject the reaction solution from the TIP head 19 into the magnetic particle liquid reagent tank 50, completing the separation of the magnetic particles and the reaction solution;
   (5) Cleaning magnetic particles:
      The X-axis screw motor 16 moves the first cleaning solution tank of the reagent strip 39 below the TIP head 19. The magnetic rod motor 3 temporarily moves the magnetic rod 5 away, and the Z-axis motor lowers the TIP head 19 into the cleaning solution. The injection pump 15 provides negative pressure to aspirate the cleaning solution, and then the injection pump 15 provides positive pressure to discharge the cleaning solution from the TIP head 19. This process is repeated for a certain period of time. Then, the injection pump 15 provides negative pressure to aspirate the cleaning solution, and the Z-axis motor lifts the TIP head 19, The magnetic rod motor 3 moves the magnetic rod 5 close to the sidewall of the TIP head 19 and slowly discharges the cleaning solution. Repeat the above steps and complete the subsequent cleaning work in the second and third cleaning solution tanks respectively:
      (6) Mix the magnetic beads and substrate solution and place them in the reaction tank 52:
      The Z-axis screw motor 25 lifts the TIP head 19, while the X-axis screw motor 16 moves the reaction tank 52 of the reagent strip 39 below the TIP head 19. The Z-axis screw motor 25 lowers the TIP head 19 into the reaction tank 52, and the injection pump 15 provides positive repulsion to inject the substrate solution into the reaction tank 52. The injection pump 15 then provides negative pressure and positive pressure several times, completing the mixing of the magnetic beads and substrate solution;
      (7) completes the detection and signal output of the reaction solution:
         The X-axis screw motor 16 moves the reaction tank 52 to the bottom of the objective lens of the optical motion detection module 9, turn on the excitation light source, and the optical detector will convert the received near-infrared fluorescence signal into an electrical signal output; The y-axis through axis motor 8 moves to move the detection module sequentially above the reaction tanks 52 of the other seven sets of reagent strips, completing the detection and signal output of the other seven sets of reaction liquids.

## Claims

1. a confocal laser technology-based fully automated immunoassay analyzer, comprising: an X-direction motion module, a reagent strip module, a Z-direction motion module, an operating arm module, a magnetic attraction module, and an optical motion detection module, wherein the X-direction motion module comprises a rubber foot, a large base plate, an X direction screw motor, a motor fixing seat, a wide guide rail, anda first connecting block, wherein the first connecting block is connected to the slider of the wide guide rail and the nut of the X-direction screw motor, so that when the X-direction screw motor rotates, The first connecting block can be moved along the sliding rail direction of the wide guide rail; the reagent strip module includes a reagent strip fixing plate, a tube head frame, a waste liquid tank, a reagent strip, and a heating rod, wherein the reagent strip is connected to the first connecting block of the fixed plate and the X-direction motion module; The Z-direction motion module comprises a connecting plate, a slant support, a vertical column, an upper fixing plate, a lower fixing plate, a Z-axis screw motor, a guide column, a sliding block, a guide column fixing seat, a connecting arm, and a shaft blocking vertical column; the z-axis screw motor connects the guide column sliding block through the connecting arm, the guide column sliding block can slide up and down along the guide column, and the shaft blocking vertical column is fixed on the lower fixing plate: the operating arm module comprises a discharging gun, a TIP withdrawing structure, an injection pump, and a PTFE pipe; The magnetic attraction module comprises a fixed plate, a magnetic attraction motor, gears, racks, linear slide rails, a magnetic attraction plate, and a cylindrical magnet, the magnetic attraction module is installed in the area below the Z-direction motion module and above the reagent strip fixed plate, the magnetic attraction motor is installed on the fixed plate, which is equipped with gears, the magnetic attraction plate is installed on the sliding block of the slide rail, the optical motion detection module includes a crossbeam, a Y-direction through-axis motor, a motor base, a connecting block, a linear slide rail, a manual fine-tuning table and a optical detector.

2. The analyzer according to claim 1, **characterized in that** one end of the discharging gun is used to load the TIP head, the other end of the discharging gun is connected to the injection pump through a PTFE tube, which can aspirate and inject the test solution.

3. The analyzer according to claim 1, **characterized in that** there are two sets of linear sliding rails installed on the fixed plate in the magnetic attraction module.

4. The analyzer according to claim 1, **characterized in that** the magnetic attraction plate is equipped with a rack and 8 circular magnets are installed at the ends.

5. The analyzer according to claim 1, **characterized in that** the motion range of the optical motion detection module covers all reagent strips on the reagent strip fixing plate.

6. The analyzer according to claim 1, **characterized in that** the optical detector is connected to the second connecting block through a manual fine-tuning table.

7. The analyzer according to claim 1, **characterized in that** the operating arm module is connected to the slider of the Z-direction motion module and can move vertically.

8. The analyzer according to claim 1, **characterized in that** the second connecting block is fixed on the sliding rail slider and connected to the motor, When the motor is operating, the second connecting block can move left and right along the sliding rail.

9. The analyzer according to claim 1, **characterized in that** the crossbeam is fixed on the column of the Z-direction motion module, which is equipped with a linear sliding rail and a Y-direction through axis motor.

10. A laser confocal technology based immune detection method, **characterized in that** the method is applicable to the immune analyzer as claimed in claim 1 and comprises the following steps:
(1) Aspirate the sample and lysates:
The Z-axis screw motor lifts the discharging gun, while the X-axis screw motor transports the sample slot below the TIP head. The Z-axis screw motor then lowers the TIP head into the sample slot,the injection pump provides negative pressure to the TIP head through the PTFE tube, and the TIP head aspirates the sample liquid; Repeat the above steps, lower the TIP head into the cracking reagent tank, and aspirate the lysates;
(2) Mixing and dilution of samples and lysates:
The X-axis screw motor moves the diluent reagent tank below the TIP head, while the Z-axis screw motor lowers the TIP head into the diluent reagent tank, the injection pump provides positive pressure to inject the sample and lysates into the diluent, while the injection pump provides negative pressure to aspirate the mixture into the TIP head, the injection pump then provides positive pressure to inject the mixture into the diluent reagent tank, repeat this process several times to complete the mixing and dilution of the sample and lysates;
(3) Mix the mixture with magnetic particle liquid:
The injection pump provides negative pressure and draws a certain volume of diluent; The Z-axis screw motor lifts the TIP head, while the X-axis screw motor moves the magnetic particle solution reagent tank below the TIP head; the Z-axis screw motor lowers the TIP head into the magnetic particle solution, and the injection pump provides positive pressure to inject the mixture of sample and lysates into the magnetic particle solution, the injection pump sequentially provides negative pressure and positive pressure several times to fully mix the aforementioned mixture;
(4) Constant temperature incubation and separation of magnetic particles from reaction solution:
Connect the power to the two heating rods and keep the incubation tank **in** a certain constant temperature environment; after incubating at a constant temperature for a certain period of time, the injection pump provides negative pressure to aspirate all the magnetic particle liquid, and the Z-direction screw motor lifts the TIP head; The magnetic rod motor drives the magnetic rod to move forward and approach the sidewall of the TIP head: after a certain period of time, the magnetic particles are completely adsorbed on the inner wall of the TIP head, The injection pump provides positive pressure to inject the reaction solution from the TIP head into the magnetic particle liquid reagent tank, completing the separation of the magnetic particles and the reaction solution;
(5) Cleaning magnetic particles:
The X-axis screw motor moves the first cleaning solution tank of the reagent strip below the TIP head, The magnetic rod motor temporarily moves the magnetic rod away, and the Z-axis motor lowers the TIP head into the cleaning solution, The injection pump provides negative pressure to aspirate the cleaning solution, and then the injection pump provides positive pressure to discharge the cleaning solution from the TIP head, This process is repeated for a certain period of time, Then, the injection pump provides negative pressure to aspirate the cleaning solution, and the Z-axis motor lifts the TIP head, The magnetic rod motor moves the magnetic rod close to the sidewall of the TIP head and slowly discharges the cleaning solution, Repeat the above steps and complete the subsequent cleaning work in the second and third cleaning solution tanks respectively:
(6) Mix the magnetic beads and substrate solution and place them in the reaction tank:
The Z-axis screw motor lifts the TIP head, while the X-axis screw motor moves the reaction tank of the reagent strip below the TIP head, The Z-axis screw motor lowers the TIP head into the reaction tank, and the injection pump provides positive repulsion to inject the substrate solution into the reaction tank, The injection pump then provides negative pressure and positive pressure several times, completing the mixing of the magnetic beads and substrate solution;
(7) completes the detection and signal output of the reaction solution:
Move the reagent strip reaction tank to the lower part of the objective lens of the optical motion detection module towards the screw motor, turn on the excitation light source, and the optical detector will convert the received near-infrared fluorescence signal into an electrical signal output; The y-axis through axis motor moves to move the detection module sequentially above the reaction tanks of the other seven sets of reagent strips, completing the detection and signal output of the other seven sets of reaction liquids,
